# EUROPEAN PATENT SPECIFICATION

(11) **EP 2 344 041 B1**
(45) Date of publication and mention of the grant of the patent: **25.12.2019**
(21) Application number: 09817338.8
(22) Date of filing: 02.10.2009
(51) Int. Cl.: A61B 6/03, A61B 6/06, A61B 6/00

(54) **METHOD AND DEVICE FOR PERFORMING COMPUTED TOMOGRAPHY X-RAY IMAGING**
VERFAHREN UND VORRICHTUNG FÜR COMPUTERTOMOGRAFISCHE RÖNTGENBILDGEBUNG
PROCÉDÉ ET DISPOSITIF POUR UNE IMAGERIE TOMOGRAPHIQUE À RAYONS X INFORMATISÉE

(30) Priority: 03.10.2008 FI 20085939
(43) Date of publication of application: 20.07.2011
(73) Proprietor: PaloDEx Group Oy, 04300 Tuusula (FI)
(72) Inventor: SIREN, Juuso, 02940 Espoo (FI); JOUHIKAINEN, Petri, 04430 Järvenpää (FI); SUURONEN, Esa, 04200 Kerava (FI); SETÄLÄ, Henri, 20660 Littoinen (FI); KALKE, Martti, 04300 Tuusula (FI)
(74) Representative: Berggren Oy, Helsinki & Oulu
(86) International application number: PCT/FI2009/050796
(87) International publication number: WO 2010/037911

(56) References cited:
- WO-A1-2006/013325
- WO-A2-2008/021671
- WO-A2-2008/021671
- JP-A- 2004 045 212
- US-A1- 2004 013 225
- US-A1- 2004 066 877
- US-A1- 2004 258 195
- US-A1- 2005 265 523
- US-A1- 2008 137 802
- US-B1- 6 246 742
- US-B1- 6 546 068

## Description

### Field of the invention

Tomographic X-ray imaging (CT, Computed Tomography) has an important role in producing three-dimensional image information of the internal structure of an object being imaged.

### Prior art

Tomographic X-ray imaging is performed by typically taking hundreds of projections around the object either by pulsing radiation or with continuous radiation. The projections are fed into a reconstruction algorithm which calculates a volumetric model on the basis of the projections taken of the imaged object and the geometry of the device.

The X-ray detectors adaptable to the prior art Cone Beam Computed Tomography, or CBCT imaging, have a large surface area and are expensive. As a result of this, CBCT devices are also expensive, and another disadvantage is that a detector with a large surface area cannot be developed so as to have as sophisticated features as a detector with a smaller surface area. A detector with a large surface area also makes more demands on the X-ray source, that is, the X-ray tube head, because the primary cone should be of good quality throughout the X-ray detector area.

As closest prior art is cited the Applicant's own earlier patent application WO 2007/020318 "X-ray imaging apparatus and X-ray imaging method for eccentric CT scanning", which discloses a method and a device for performing tomographic cone beam X-ray imaging in such a way that the rotation centre between the X-ray source and the X-ray detector is arranged to move along a predetermined route as the support means rotate around the axis of rotation, whereupon the field of view can be enlarged with the same exposure parameters, or alternatively, the radiation dose may be reduced while the field of view remains the same in comparison to a case where the said rotation centre is connected to the axis of rotation. The aim of the imaging method disclosed in the publication WO 2007/020318 is to improve the utilisation of the field of view, so that a larger field of view (FOV) will be obtained by other means than increasing the surface area of the X-ray detector or by changing the geometric parameters of the device, for example the SID and the coefficient of enlargement. The problem with the said implementation is, however, an increase in the size of the device, which prevents the achievement of the cost advantage that would be achieved with a somewhat smaller X-ray detector. Another problem is that good results in reducing the amount of radiation to which the patient is subjected are not achieved without the image quality deteriorating.

There are devices on the market which perform so-called offset scanning, for example Scanora 3D manufactured by Soredex, by means of which it has been possible to enlarge the field of view (FOV). The disadvantage of offset scanning is that 360 degree scanning is required, from which thus follows an increase in the size of the device.

As prior art is also cited the patent application FI20070768 (J. Morita Manufacturing Corporation), which discloses a device for performing X-ray imaging, in which device it may be possible to choose between different forms of imaging, such as panoramic X-ray imaging or offset scanning CT X-ray imaging. Devices of this type comprise equipment for implementing various forms or imaging, which increases their manufacturing costs. The disadvantages of the device implementations disclosed in the publication FI20070768 are the large size of the device and that good results in reducing the amount of radiation to which the patient is subjected are not achieved without the image quality deteriorating.

The publication US2005265523 discloses offset imaging methods and combining data imaged with different offsets into three-dimensional data. It does not disclose the use of forward scanning imaging cycles for performing imaging.

The publication WO2008021671 discloses offset imaging configurations. According to claims 19 and 20, for example, data imaged with different offsets is processed as separate imaging processes and only the three-dimensional data is combined.

### Brief description of the invention

The aim of the invention is to provide a computed tomography X-ray device by means of which, for example, tomographic X-ray imaging can be performed with smaller X-ray detectors than before, without having to increase the overall size of the imaging device, reducing the manoeuvring area required by the X-ray detector and the X-ray source, and diversifying the image information obtained from X-ray imaging. This is achieved by means of a method for performing computed tomography, in which method X-radiation is produced with an X-ray source, which is collimated by means of a collimator to the object being imaged and the X-radiation that has been transmitted through the object is received with an X-ray detector to produce image information of the object being imaged and the said X-ray imaging is performed from different imaging angles by moving the X-ray source and the X-ray detector with respect to the object being imaged. The method utilises the offset between the centreline between the X-ray source and the radiation receiving means and the rotation centre in such a way that by using at least one X-ray imaging offset, X-ray imaging of the object is performed to produce image information, the X-ray imaging offset is changed to produce each, at least one, subsequent X-ray imaging offset, by using at least one offset of the subsequent X-ray imaging is performed at least one subsequent X-ray imaging of the object to produce at least one subsequent image information and combining the said image information to produce three-dimensional image information of the object.

The invention also relates to a device for performing computed tomography, comprising an X-ray source for producing X-radiation, a collimator for collimating X-radiation to the object being imaged, an X-ray detector for receiving the X-radiation that has been transmitted through the object to produce image information of the object being imaged and support means for supporting the X-ray source and the X-ray detector, at least during imaging in positions on opposite sides of the object, the said support means being connected to the frame part of the device so as to rotate around the axis of rotation. The device comprises actuator means for rotating the support means around the axis of rotation to perform the said X-ray imaging from different imaging angles by moving the X-ray source and X-ray detector with respect to the object being imaged by utilising the offset between the centreline between the X-ray source and the radiation receiving means and the rotation centre by using at least one X-ray imaging phase offset when performing the X-ray imaging phase of the object to produce image information, positioning means for changing the offset to the offset of at least one subsequent X-ray imaging offset for at least one subsequent X-ray imaging phase, the said actuator means for performing at least one subsequent X-ray imaging phase stage by using the said offset of the at least one subsequent X-ray imaging phase in performing at least one subsequent X-ray imaging phase to produce at least one subsequent image information and an image-processing unit for combining the said image information to produce three-dimensional image information of the object.

The invention is based on the fact that in the X-ray imaging of the object is utilised the changing of the offset between the centreline between the X-ray source and the radiation receiving means and the rotation centre in the middle of the imaging session, either during the imaging phases or between imaging phases, so that the X-ray imaging of the object is divided into imaging phases in which the object is imaged with different offsets. The image information produced in the imaging phases using different offsets is combined by image processing to produce three-dimensional image information of the object.

By means of the invention is achieved successful tomographic X-ray imaging with smaller X-ray detectors than before, without having to increase the overall size of the imaging device. One advantage of the invention is that in the X-ray imaging device, the manoeuvring area required by the X-ray detector and the X-ray source can be reduced, because the X-ray detector and the X-ray source do not have to turn a full 360 degrees, but an imaging area of, for example, approximately 180 degrees will suffice. This makes possible, for example, CT imaging with a conventional panoramic device, in which the X-ray detector and the X-ray source cannot together rotate, for example, 360 degrees. A further advantage is that the image information obtained from X-ray imaging becomes more versatile. Especially compared with prior art scanning offset imaging, when performed in accordance with the invention, the X-radiation is directed at the head of a patient, who is the object of dental arch imaging, from behind, in which case organs or tissues susceptible to radiation receive a less effective dose of radiation due to the fact that a significant part of the radiation has already been absorbed in the hard tissue (bone) preceding it. By selecting the incoming direction of the radiation, the disadvantages of radiation can be influenced also in other than dental imaging if the imaging area is smaller than a full 360 degrees.

Compared with prior art CBCT devices, which are used for performing X-ray imaging by scanning 180 or 360 degrees symmetrically, an advantage of the implementation according to the invention is the possibility to use the X-ray source with a smaller anode angle than in prior art implementations. This means that a narrower cone of rays is formed, in which case the amount of scatter radiation is small.

### List of figures

- Figure 1: shows diagrammatically an X-ray imaging device according to the invention which is suitable for implementing the method according to the invention.
- Figure 2: shows symmetrical scanning according to the prior art.
- Figure 3: shows prior art offset scanning as a cross-section of the cylinder plane.
- Figure 4: illustrates a first preferred embodiment of the invention.
- Figure 5: illustrates a second preferred embodiment of the invention.
- Figure 6: illustrates a preferred imaging phase of the method according to the invention.
- Figure 7: illustrates alternative methods of implementation of the first preferred embodiment of the invention.
- Figure 8: illustrates alternative methods of implementation of the second preferred embodiment of the invention.

### Detailed description of the invention

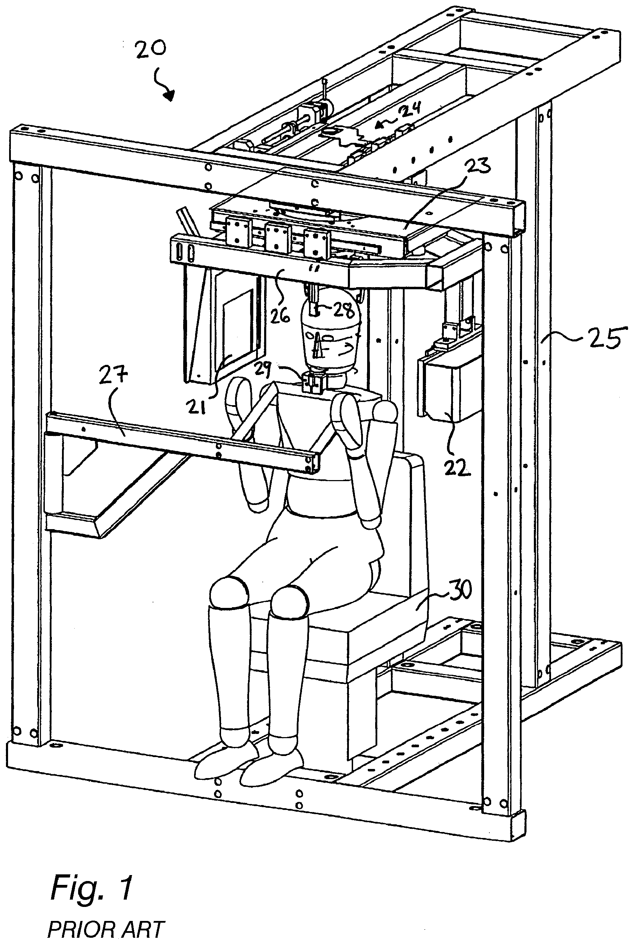

Figure 1 shows one example of a dental and/or head area imaging device 20 suitable for implementing the method according to the invention, comprising a frame part 25, on which is supported a rotating part 23 with support means 26, at the first end of which is at least one X-ray source 22 for producing X-radiation, and on the opposite side, at least during imaging, as radiation receiving means is at least one X-ray detector 21, and the object being imaged is positioned in the area between the X-ray source 22 and the X-ray detector 21. If it is desirable for the patient to sit during imaging, there may be a seat 30 in the device for the person being imaged. The device further comprises adjustable support means 27 and positioning means 28, 29 for positioning the object being imaged correctly with respect to the X-ray source 22 and the X-ray detector 21 and/or positioning means for positioning the rotating part with respect to the object. The X-radiation produced by the X-ray source is collimated by means of a collimator into a cone beam of rays to the object being imaged and the X-radiation that has been transmitted through the object is received with an X-ray detector to produce image information of the object being imaged. The cone of rays is usually pyramid-shaped when a square X-ray detector and collimator opening are used, and the cone of rays is conical when a round X-ray detector and collimator opening are used. The cone of rays may also be, for example, a crescent-shaped cone or the like. The method according to the invention may also be implemented by using more than one X-ray source.

The device according to the invention shown in Figure 1 comprises positioning means, that is, displacement and/or rotating means for changing the positioning of the X-ray source, the collimator and the X-ray detector with respect to one another and actuator means for rotating the support means partly or completely around the axis of rotation to perform the said X-ray imaging from different imaging angles by moving the X-ray source and X-ray detector with respect to the rotation centre between them and the object being imaged.

By the positioning means, the imaging method can be changed into symmetrical imaging or offset imaging. By using the positioning and actuator means in question, the method of imaging can be selected to change from symmetrical imaging to offset imaging of vice versa even during imaging. Offset imaging may also be partial, in which case the central area is imaged symmetrically with a 360 degree combined angle of projection and the edge areas with a 180 degree offset so that during the imaging phase, radiation will be emitted only from one side to each point being imaged in the edge area. The actuator means comprise a rotating part 23 by means of which the support means are arranged to rotate around the axis of rotation, the said axis of rotation passing through the field of view of the object being imaged. A mechanical rotation centre is in question in this case. When a virtual rotation centre is in question, the said axis of rotation moves in a horizontal x-y direction, which means that it may move through the field of view of the object being imaged either intermittently, continuously or not at all. The actuator means also comprise a control system for controlling and coordinating the movements of the device parts, for example the positioning means, when carrying out the different stages of the X-ray imaging process of the invention. In front of the X-ray source, before the object being imaged, is a collimation system comprising at least one collimator, which is moved by means of the control system, synchronically with the movement of the X-ray detector with respect to the X-ray source.

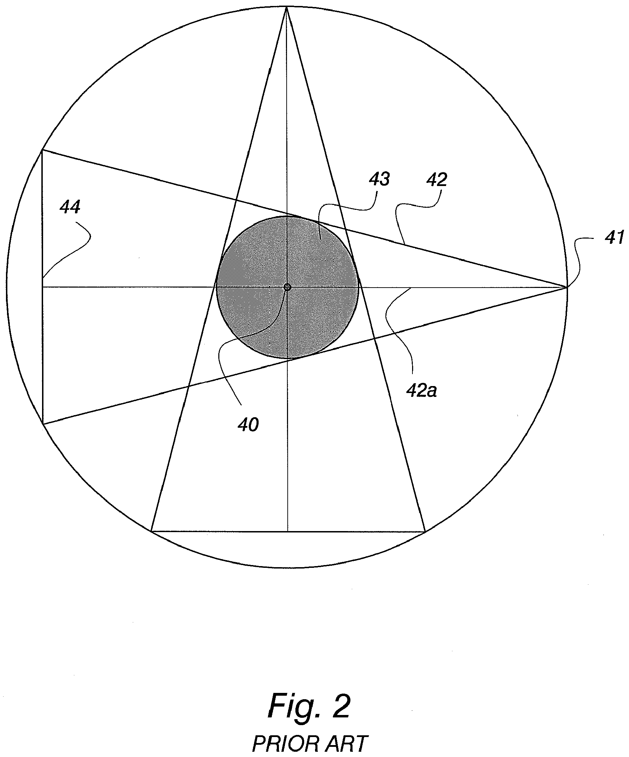

Figure 2 shows symmetrical scanning according to the prior art. The whole object 43 being imaged is irradiated by means of the X-ray source 41. The radiation transmitted through the object is detected by the X-ray detector 44. Imaging is performed by rotating the X-ray source 41 and the detector 44 around the rotation centre 40 with respect to the object being imaged. The detector 44 and the radiation source 41 may be fixed to a C-arm or a slip ring and the detector and radiation source will rotate around the object during imaging. It is also possible to rotate the object being imaged. The imaging produces a plurality of 2D-projections of the object 43. Of the data on the projections is formed the final computed tomography image. With the symmetrical geometry according to Figure 2, 180 degree rotation produces image data of the surroundings of each point being imaged over an area of 360 degrees.

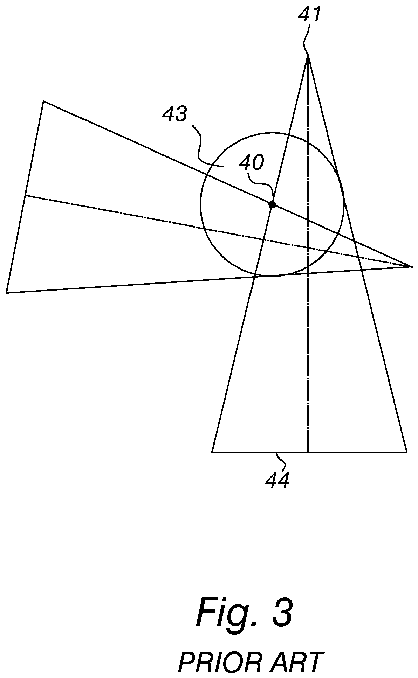

Figure 3 shows the principle of offset scanning according to the prior art. The image area of the detector is on the side, in line with the radiation source and the rotation centre. In this example, the whole object 43 can be modelled with one 360 degree rotation, and half the width of the imaging area of the detector of Figure 2 suffices as the width of the imaging area of the detector. With the geometry of Figure 3, image data for 360 degrees cannot be obtained with a 180 degree rotation, but a full 360 degree cycle is required.

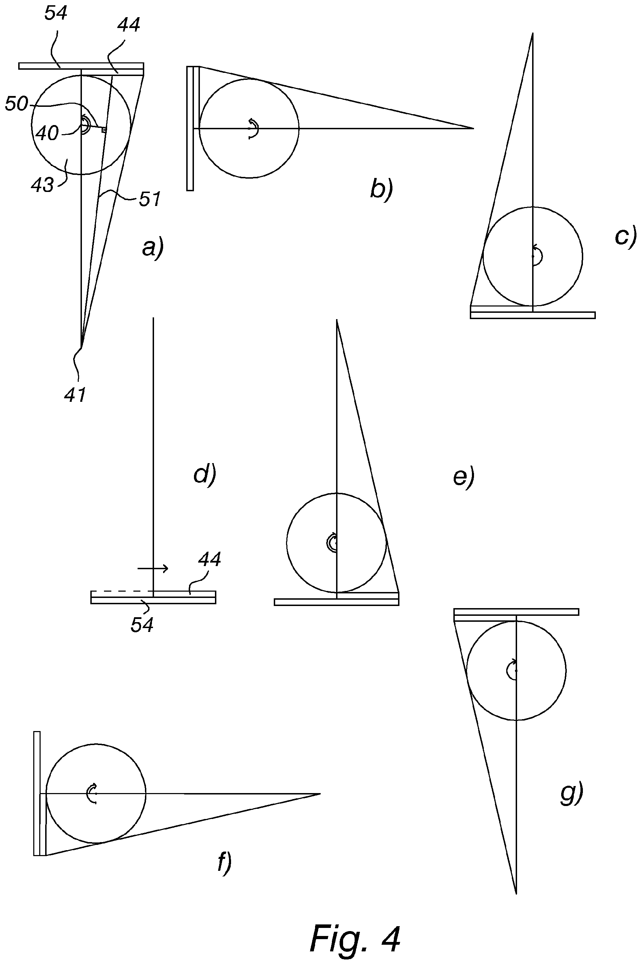

Figures 4 to 8 show imaging methods or arrangements according to the invention.

The device according to the invention preferably also comprises means with which symmetrical imaging, prior art offset imaging, or offset imaging according to the invention can be selected as the imaging method. The imaging method may be selected, for example, by means of a switch, through the control system, or otherwise. The device according to the invention may be a combined device, for example a combined device intended for CT and panoramic imaging.

In the method according to the invention and in its more preferred implementation, the centreline between the X-ray source and the radiation receiving means is offset from the rotation centre on the axis of rotation during imaging, between the imaging phases or during them. The offset can be defined as the shortest distance of the centreline between the X-ray source and the radiation receiving means from the rotation centre, that is, the magnitude of the offset of the centreline between the X-ray source and the radiation receiving means. In addition to the magnitude of the offset, the offset also has a direction, which may be marked with a sign. Offset can be set to initial value prior imaging. In this description, maximal offset or the maximal displacement of the offset refers to the largest offset which does not leave an unimaged strip between the area imaged in the adjacent imaging phase, or an unimaged empty area around the rotation centre, nor on the other hand expose the object twice unnecessarily.

The rotation centre may be mechanical or virtual and the rotation centre may also move during the imaging session. A virtual rotation centre is obtained, for example, by moving the mechanical rotation centre along a circular path, whereupon the virtual rotation centre is formed in the centre of the said circular path. Non-circular scanning may be produced, for instance, by moving the source and the detector along a path deviating from a circular path, for example an elliptic path. The offset can then be determined in the appropriate manner.

The sign of the offset is determined by the side of the axis of rotation on which the centreline is when viewed from the X-ray source to the detector. In the arrangements according to Figures 3 to 6, when the centreline between the sensor and the source is on the left side of the axis of rotation, when viewed from the source, the sign could be, for example plus, and thus in the case of the centreline being on the right side, the sign of the offset is minus.

In implementing the method according to the invention, a scanning imaging movement is used, where the X-ray source and the X-ray detector are moved synchronically with respect to one another and projections of the object are taken at desired imaging angle intervals, either during the movement or by stopping the X-ray source and/or X-ray detector momentarily for taking each projection.

The method according to the invention is implemented by using at least one X-ray imaging phase offset in performing X-ray imaging of the object to produce image information. After this, the positioning means are used to change the positioning of the X-ray source, the collimator and/or the X-ray detector with respect to one another to produce an offset differing from the offset of the previous imaging phase for at least one subsequent X-ray phase. By using the changed positioning, the offset differing from the previous offset is used to perform at least one subsequent X-ray imaging phase of the object to produce at least one subsequent image information. The said image information is combined in image processing carried out by using an image processing unit, that is, a computer to produce three-dimensional image information on the object.

According to the invention, the imaging may also be carried out by means of one scan. In such a case, the offset is changed during scanning, which means that projections imaged with different offsets possibly become imaged into one or more scanning files. The offset can, in that case, be changed continuously or stepwise also during a single scan. Also in this case, the object is imaged several times with different offsets during one imaging session, whereupon several imaging phases of the object are apparently imaged in one scanning phase, so that information on the object is obtained with several different offsets by imaging during the same imaging session. In this case, the imaging phases take place within the same scanning session and the data from the imaging phases may end up in one scanning file. By changing the offset according to the invention, additional information on the object is obtained during imaging by imaging a new area with a new offset, in which case the field of view is extended. Alternatively, the same area may be imaged at least partly, whereupon after changing the offset, imaging will take place from a different direction and more information will be obtained of the area. Therefore, by means of the method according to the invention, due to the change of offset, more information is obtained on the object than would otherwise be possible with the same imaging apparatus.

The offset may be changed, for example, stepwise or in a slidingly. The three-dimensional imaging areas of successive imaging phases may partially intersect one another. Thus, in the method according to the invention, the surroundings of at least some of the areas of the object are imaged with different offsets to extend the imaging area again or, after the changing of the offset, an area of the object is imaged from a different direction to obtain additional information on the object. The fields of view of successive imaging phases may form, for example, a spiral pattern on the cross-sectional plane perpendicular to the axis of rotation. The spiral pattern approaches or withdraws with respect to the rotation centre and it may change its direction. The angle of the axis of rotation or the position of the axis of rotation may, in addition, be changed during imaging. Successive areas imaged in the imaging phases may also be separate annular areas and should the position or angle of the axis of rotation or virtual axis of rotation be changed, the areas exposed during the imaging phase may deviate from circular arcs. In that case, the difference in offset is understood as a displacement with respect to the path used in the previous imaging phase or first imaging phase.

Figures 4a to 4g illustrate the first preferred embodiment of the invention, where the first X-ray imaging of the object is performed by first scanning an approximately 180 degree image angle area with an essentially maximal first X-ray imaging offset, that is, a so-called full offset, whereupon 180 degree tomographic imaging is produced in several, for example, hundreds of pieces of projection information taken on the first side of the object being imaged by scanning imaging movement. The X-ray detector is then displaced to the other side of the rotation centre to obtain an essentially maximal second X-ray imaging offset, that is, a full offset of a second imaging direction, and the cone of rays is collimated to the displaced X-ray detector.

Stage 4a in the Figures shows the starting situation of an imaging phase before the first scanning of the imaging phase. The offset 50 of the detector 44 is half the width of the imaging area of the detector. In this case, the edge of the beam of rays emitted from the X-ray source intersects the rotation centre 40. This is a full or maximal offset, which gives a maximal imaging area without an empty area in the middle or a partly symmetrical scanned area in the middle. The detector is mounted movably on the positioning means 54. At stage 4b, the scanning of the first imaging phase has proceeded 90 degrees from the starting situation. At stage 4c, the scanning of the first imaging phase has proceeded a full 180 degrees, and the first imaging phase has ended.

At the following stage 4d, the detector 44 is moved by the positioning means 54 to the offset position with the opposite sign, to the other side of the line determined by the rotation centre 40 and the X-ray source.

In this example, the scanning of the second imaging phase is performed in backwards direction so that the initial position of the second imaging phase is described in Figure 4e, the halfway position of scanning in Figure 4f and the final position in Figure 4g. It is possible to scan both imaging phases in the same direction, in which case the imaging means are moved in connection with moving the detector to a position described in Figure 4g, and the scanning of the second imaging phase takes place via the position in Figure 4f to the position in 4e. The scanning of both imaging phases thus takes place in the same direction. This may be advantageous for calibration or repeatability.

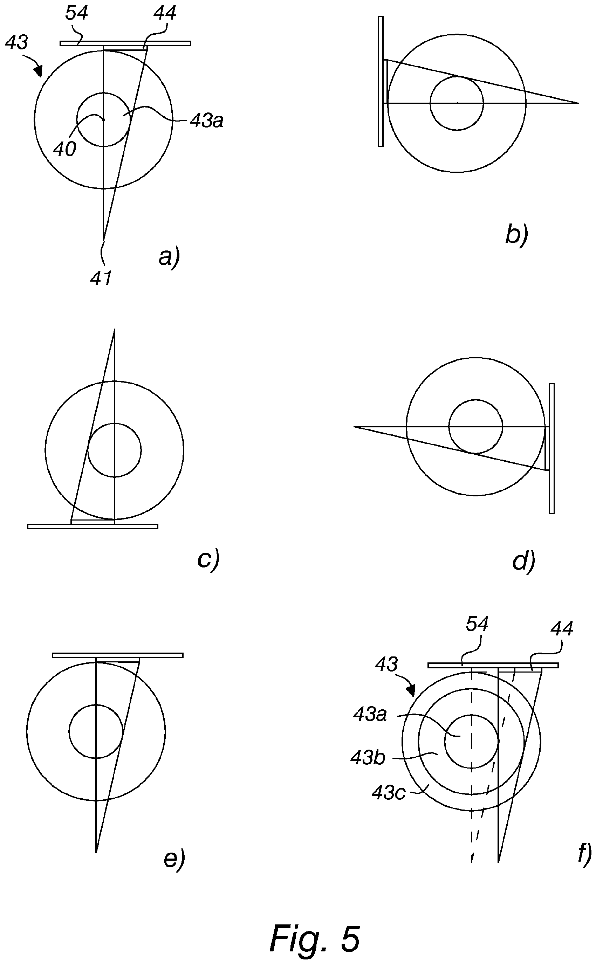

It should be noted that if the rotation centre 40 does not belong within the desired imaging area, the offset may be larger than described above. The phases described above may also be only a part of the overall imaging process, in which case the offset is increased at the next stage, for example, from a 0.5 detector imaging width preferably by almost one imaging width further from the imaging width of approximately 1.5. In such a case, a larger area than described above is scanned during the third and fourth imaging phases. In the geometry of Figure 4, the position of the detector is too close to the object being imaged, and extending the offset would require, for example, the imaging geometry shown in Figure 5.

At stage 4d, the cone of rays is collimated to the moved X-ray detector by changing the position of the collimator and/or the X-ray source so that the collimator or the combination of collimator and X-ray source is moved or turned so that the beam of rays is directed at the moved X-ray detector. The second X-ray imaging phase of the object is carried out by scanning the said about 180 degree imaging angle area again, whereupon the 180 degree tomographic imaging is produced on the other side of the object being imaged. Thus, with an imaging apparatus which turns about 180 degrees is obtained imaging information on the whole area of the object at an imaging angle of 360 degrees, and the 360 degree scanning movement around the object, as required in the method of Figure 3, is avoided.

The most advantageous way of implementing the first preferred embodiment of the invention is to perform the said first and second X-ray imaging from an imaging angle area which is slightly, for example 1 to 10 degrees, more than 180 degrees, or even more, which means that less estimative calculation information is required in processing the image information and thus less capacity is required of the software used than when performing imaging in smaller imaging angle areas.

The first preferred embodiment of the invention can also be realised in such a way that the X-ray detector is moved to the other side of the virtual rotation centre.

One possible method of implementing the first preferred embodiment of the invention is, for example when using a narrower X-ray detector, to displace the X-ray detector further after the first 180 degree scanning in order to change the offset at least once, or the displacement may possibly also be continuous. In such a case, however, when imaging an imaging area of 360 degrees with a 180 degree device, at some point the centreline between the X-ray detector and the source is moved to the other side of the mechanical or virtual rotation centre in order to change the sign of the offset, and the corresponding scanning stages are also performed for the offsets with the opposite sign. Several advancing scanning cycles are then performed first using offset values with the same sign, whereupon each scanning cycle produces data on an arch-shaped area. The forward moving imaging cycles can be scanned by moving the sensor and detector in either direction, because the direction of rotation of the scanning is of no significance from the point of view of the scanning. The direction of displacement of the imaging cycles may be towards the centre or away from it. It is also possible to image the arch-shaped areas in an arbitrary order, although this does not bring any advantage to performing the imaging. Once, for example, a 180 degree area has been imaged by scanning several arcs with a narrow sensor, the imaging means are moved so that imaging can also be performed using an offset with the opposite sign. Therefore, according to the invention, a larger volume can be imaged with a narrow sensor by moving the imaging area stepwise or slidingly closer to or further away from the centre of curvature.

The projection information produced in the said first and second X-ray imaging are combined by image processing carried out by means of an image processing unit to produce three-dimensional image information of the object. This is done, for example, by transferring the projection information and the geometric information relating to it, such as the coordinates of the positions of the corner points of the X-ray detector and the focus of the X-ray source relating to each piece of projection information into a calculation algorithm, by means of which is calculated a volumetric model of the object imaged. To the X-ray detector is connected a projection capturing system, which may be a part of the control system comprised by the actuator means or also separate. The function of the capturing system is to store the projection information and to transfer it to the calculation algorithm.

In the first preferred embodiment of the invention, previously obtained image information can also be utilised as input information for the calculation algorithm in producing three-dimensional image information of the object. This is particularly useful in performing scanning X-ray imaging in an imaging angle area of less than 180 degrees. It is also possible to utilise other calculation-technical methods to improve image quality, which methods make it possible to use a less than 180 degree scanning imaging angle area. As an example could be mentioned the utilisation of tomosynthetically produced image information in blurred form, that is, externally of the sharp layer, calculatorily in a reconstruction algorithm for developing image quality.

Instead of scanning 180 degrees twice, the imaging may be performed by first imaging, for example, an imaging angle area of 270 degrees, transferring the offset to the other side of the rotation centre, positioning the imaging means for a new scanning so that the 90 degree imaging angle area lacking from 360 degrees is scanned. The entire 360 degree image angle area can thus be covered with two scans together amounting to a 360 degree image angle, instead of 180 degrees. The aforedescribed two scans with essentially two 180 degree scans is thus the simplest, but not the only, option according to the invention.

It is also possible to carry out the first preferred embodiment of the invention in such a way that the scanning is performed in a narrower area, for example 90 degrees, and at some stage between scans, the object being imaged is turned by the required lacking number of degrees, whereupon corresponding image information is obtained as described above.

The first preferred embodiment of the invention is also suitable for 360 degree imaging. In that case, instead of approximately 180 degrees, an imaging angle of approximately 360 degrees is scanned twice in the manner described above with offsets of opposite signs. This is useful in some special cases where image information from a particular area of the object is required from various angles.

Figure 5 shows a second preferred embodiment of the invention.

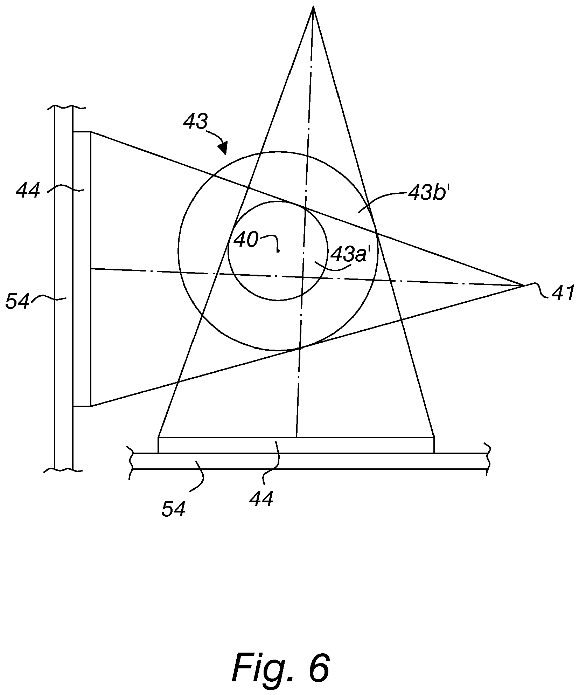

Figure 5a shows a stage at which the second embodiment is depicted in the initial position before the first X-ray imaging phase. The straight line determined by the source 41 and the rotation centre hits the edge of the detector. This is the maximal offset position if the whole object 43 is the object of interest. Should the surroundings of the rotation centre not be of interest, a larger offset may be used, in which case the centre of the object is not imaged. It is also possible to use incomplete offset, in which case the area of the centre is imaged by 360 from two directions, this starting position also being shown in Figure 6.

The first imaging phase scans the object through stages 5a, 5b, 5c, 5d and 5e. This gives the first phase imaging data that covers area 43a.

After the first imaging phase, the X-ray source 41 and the detector 44 are moved according to Figure 5f in such a way that the offset measured from the centre 40 increases so that the edge of the area 43b to be scanned next hits or slightly overlaps the previously imaged area 43a.

It should be noted that in connection with the moving according to Figure 5f, the offset value cannot be determined directly by means of the displacement projected to the level of the detector by moving the detector by its width, as could be done when rotating with respect to the source. In accordance with Figure 5f, the parallel move away from the centre determines the change in the offset. The rotation and the parallel move or their combinations are, for example, possible ways of providing offset. The offset may also be provided by means of other displacements with which the line determined by the detector 44 and the X-ray source 41 moves further away from the rotation centre 40. The mutual distance or alignment of the X-ray source and the detector can be changed at the same time.

In Figure 5f, the parallel move and rotation with respect to the source to change the offset result in slightly different irradiation and imaging accuracy in different parts of the object. In addition, the data from the different scanning phases must be combined in a different manner. The rotation of the source and the parallel move of the sensor make it possible to combine the data in 2D format before 3D reconstruction, but a greater degree of freedom to choose the imaging directions for different areas is achieved by other methods.

After stage 5f is performed the scanning of the second imaging phase, during which is obtained image information for the whole area 43b. After the second imaging phase, the imaging means can be moved once more to a third offset position, after which the remaining area 43c can be scanned in a third imaging phase.

In this way is produced, for example by means of two imaging phases, an almost double width for the cylinder shape being modelled compared with the 360 degree offset scanning according to the prior art.

In the second preferred embodiment of the invention, the device is designed in such a way that the offset can be changed more than twice or three times without limiting the scanning to one cycle and the object can be imaged in several scanning cycles, increasing (or reducing) the offset for each cycle. Scanning may also be continuous and by one, for example spiral, scan can be imaged several imaging phases according to the invention with different offsets by changing the offset between the cycles either stepwise or continuously. The said device is preferably realised in such a way that there are no limitations on the forward rotation of rotating parts. This may be done, for example, by using slip rings for power transmission between the rotator and the frame to achieve uniform and rapid scanning.

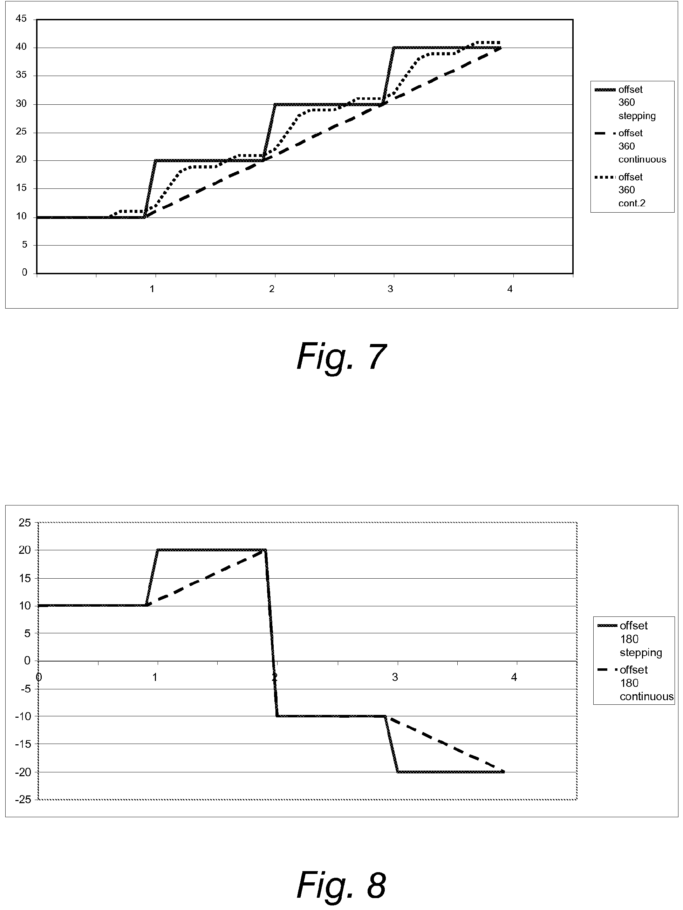

Figure 7 shows possible ways of changing the offset in scanning the successive imaging phases according to the second embodiment. The vertical axis in Figure 7 shows the amount of offset. Should it be desirable to image the object to be imaged as a whole, the numerical value on the vertical axis should in this diagram be understood as the distance of the furthest edge of the beam of rays passing the centre from the rotation centre, and the width of the beam of rays is 10 units as seen from the centre at the closest point. The offset defined with respect to the centreline of the sensor is, therefore, 5 units smaller with respect to the absolute values of the vertical axis of Figures 7 and 8.

The unit of the horizontal axis in Figure 7 are the imaging cycles. The unbroken line depicts stepwise displacement which produces a point of discontinuity in the data and possibly forces the rotational movement to be stopped in between scans. These disadvantages can be diminished by scanning a cycle exceeding 360 degrees at each imaging phase, so that each imaging phase will produce a full round of data before the stepwise displacement. In this case the steps will, therefore, occur in different angles of rotation in different imaging phases and partial double-exposure will take place. During the shifting of the offset, the X-ray source may also be switched off, thus avoiding unnecessary double-exposure during the displacement.

The broken line in Figure 7 depicts continuous offset displacement. It produces partially overlapping image data, but on the other hand does not require stopping. The dotted line in Figure 7 depicts an intermediate form between the previous two.

The offset is increased, for example, by slightly less than the width imaged by the X-ray detector for each of the following scanning cycles, which means that the scanning cycles overlap somewhat. In this way is formed a multiple width for the cylindrical form of the object of imaging being modelled compared with the prior art 360 degree offset scanning. Should there be limitations on the forward rotation of the scanning, the scanning can be carried out, for example, in such a way that after the 360 degree scan, the next imaging phase is scanned in the other direction.

Figure 8 depicts different ways of changing the offset in connection with 180 degree scanning. Also in Figure 8, the line depicts the distance of the outer edge of the area being imaged from the rotation centre; in the above defined manner the offset would be 5 units smaller in absolute value if the imaging width was 10 units. The continuous line depicts stepwise offset displacement between 180 degree scans. The broken line depicts sliding offset displacement. At point of time 2, the offset is changed after two 180 degree scanning stages to the other side of the rotation centre, that is, the sign of the offset changes.

In one preferred embodiment of the invention, for example the 360 or 180 degree scans of the centre may be carried out by symmetrical scanning instead of offset scanning and the scans of the outer layers may be carried out by offset scanning.

Another alternative implementation according to the second preferred embodiment of the invention is that the first scanning cycle is performed by using so-called full offset as offset, after which the offset is increased preferably, for example, by the width of the X-ray detector for each subsequent scanning cycle. In this way, the projection information produced in each scanning cycle can be widened by the offset as the path of the projections taken increases by rotating perpendicularly when proceeding outwards from the centre. This makes it possible to image a large volume to be imaged, which is the object, with a narrow X-ray detector, even with a panoramic detector, as long as a sufficient number of scanning cycles is performed. The offset can be increased by the positioning means, for example, in a stepwise, evenly sliding or wavily sliding manner in accordance with Figure 7. In a typical implementation, the different layers intersect one another slightly to give a uniform volume and so that no empty areas or strips remain.

In a third preferred embodiment of the invention, the device according to the invention is used to implement scanning X-ray imaging which utilises adjustable offset. It is thus possible to choose whether to perform symmetrical scanning or full offset scanning or a combination of the said imaging forms. The device comprises positioning means by which the offset is, for example, slidingly adjustable and thus the choice between performing symmetrical or offset scanning can be made before scanning, during the imaging session.

The third preferred embodiment of the invention may be carried out, for example, in such a way that in order to reduce the radiation dose, the object is irradiated so that the object to be imaged most accurately is of the symmetrical scanning volume in the centre and on the edges the object is of the offset scanning volume, which is not subjected to radiation during about half of the imaging time. In a volume model calculated with a calculation algorithm, the difference between the volumes of the central and edge areas is shown in noise levels, because the volume of symmetrical scanning is subjected to approximately twice the amount of radiation compared to the larger volume of offset scanning. In other respects, the third preferred embodiment of the invention may comprise the corresponding things as the first and second preferred embodiment.

The reconstruction of 3D image information may be carried out either with a specifically designed algorithm or, at its simplest, using conventional algorithms so that "adjacent" projections taken from the same imaging angle in pre-processing are joined into one wide image either by means of mere geometric positioning or by means of more intelligent algorithms intended for joining images. The projections thus obtained are fed into the 3D algorithm for processing, in order to produce three-dimensional image information of the imaged object. The said image algorithm does not have to be aware whether the projections are produced in one or more scanning cycles. Partial reconstructions of data from different imaging cycles may also be produced, in which case in the final reconstruction is preferably still utilised the data remaining outside the partial reconstructions.

In the embodiments of the invention, the positioning means for changing the offset may also comprise the following implementations: 1) the X-ray source, collimator and X-ray detector are positioned fixedly with respect to one another and move as one piece in relation to the rotator and/or the object. 2) The X-ray source is located fixedly in place and the X-ray detector and collimator perform synchronised movements with respect to one another to change the offset. 3) By moving the rotation centre mechanically with respect to the frame between the X-ray source and the detector. 4) By moving the rotation centre along a spiral path using x and y movements. The movement according to point 1) can be carried out as rotational movement with respect to the X-ray source or detector, or as parallel movement with respect to both or as imaged movement of a combination of these. It is also possible to change the distance between the detector and the X-ray source. The source and the detector may be separately controllable.

In the different embodiments of the invention, the offset can be changed during the imaging sessions between different imaging phases. Symmetrical imaging may also be changed to offset imaging during an imaging session.

Figure 6 shows a partly symmetrical imaging arrangement. If the imaging area of interest is in the middle of the object 43 being imaged, the area 43a' can be imaged more accurately with symmetrical imaging and the area 43b' remaining outside is subjected to less radiation. This imaging arrangement can be used during one imaging phase in the method according to the invention, in which case at the following imaging phase would be scanned, for example, an area outside the object 43 or re-scanned an unsymmetrically scanned area.

In the embodiments according to the invention, the said rotation centre may be a mechanical rotation centre between the X-ray source and the X-ray detector, or a so-called virtually realised centre, in which case the position of the mechanical rotation centre is changed between taking the projections. In the most preferred embodiment, the rotation centre is mechanical and the image information obtained from scanning is cylindrical. When the rotation centre is virtual, the image information obtained from scanning may also be of a different shape than cylindrical.

Although the invention is described with reference to the Figures in the above specification, the invention is, however, not limited to the description and Figures, but the invention may be varied within the scope of the appended claims.

## Claims

1. A method for performing computed tomography, in which method
X- radiation is produced with an X-ray source (41), which is collimated into a cone beam by means of a collimation system comprising at least one collimator to an object (43) being imaged, and the X-radiation that has been transmitted through the object is received with an X-ray detector (44) to produce image information of the object being imaged, said detector having a width and imaging area, and
X-ray imaging is performed from different imaging angles by moving the X-ray source and the X-ray detector with respect to the object being imaged by following a scanning imaging movement where the X-ray source and the X-ray detector are moved synchronically with respect to one another, a control system moves the collimator for the collimation system synchronically with the movement of the X-ray detector with respect to the X-ray source and projections of the object are taken at desired imaging angle intervals,
in which method:
a first imaging phase is performed by scanning at least a part of the object to be imaged by following a first arc of a first rotating movement to produce first image information, said first rotating movement being a rotation about a mechanical rotation centre, the centre being in a horizontal x-y plane perpendicular to an axis of rotation, said first rotating movement having a first offset where the offset (50) is defined with respect to a centreline (51) of the detector, the offset being a shortest distance, of the said centreline between the X-ray source and the X-ray detector, from the said mechanical rotation centre,
the offset of the imaging is changed between imaging phases,
at least one other imaging phase is performed with at least one changed offset (50) to produce second image information of at least part of the object, wherein the mechanical rotation centre in the other imaging phase is moved in the plane relative to the centre of the first rotating movement of the first imaging phase, and
projection information, which was produced in the first phase and the other phase, is combined by image processing carried out by means of an image processing unit for producing three-dimensional image information of the object.

2. A method as claimed in claim 1, wherein a first or last scanning imaging phase is carried out by using as offset a predetermined offset, for example a full or no offset, that is, symmetrical offset, after or before which the offset is changed essentially by a width of an imaging area of the X-ray detector for each subsequent imaging phase, as a path of projections taken increases or decreases on a imaging plane in a form that is, for example, spiral, meandering, or a separate arc with respect to the centre.

3. A method as claimed in claim 1 or 2, wherein a positioning of the X-ray source, the collimator, and/or the X-ray detector is changed with respect to one another between the X-ray imaging phases by displacing the X-ray detector to a new position to provide a next X-ray imaging offset and by directing the radiation at the displaced X-ray detector by changing a positioning of the collimator and/or the X-ray source.

4. A method as claimed in any of the claims 1-3, wherein is used adjustable offset setting for choosing an irradiation area of the scanning X-ray imaging.

5. A method as claimed in any of the claims 1-4, wherein data collected during the scanning cycles is collected together before 3D reconstruction.

6. A method as claimed in any of the claims 1-5, wherein partial reconstructions, which are utilised in a final reconstruction, are produced of the data collected during the scanning cycles before the final 3D reconstruction.

7. A device for performing computed tomography, comprising
an X-ray source (41) for producing X-radiation,
a collimation system comprising at least one collimator for collimating X-radiation to an object (43) being imaged,
an X-ray detector (44) for receiving the X-radiation that has been transmitted through the object to produce image information of the object being imaged, said detector having a width and imaging area,
support means (54) for supporting the X-ray source and the X-ray detector during imaging in positions on opposite sides of the object,
actuator means for rotating the support means, and
positioning means (54) for changing an X-ray imaging offset (50),
which device is adapted to
move the X-ray source and the X-ray detector with respect to the object being imaged by following a scanning imaging movement where the X-ray source and the X-ray detector are moved synchronically with respect to one another, a control system moves the collimator for the collimation system synchronically with the movement of the X-ray detector with respect to the X-ray source and projections of the object are taken at desired imaging angle intervals,
perform a first imaging phase by scanning at least a part of the object to be imaged by following a first arc of a first rotating movement to produce first image information, said first rotating movement being a rotation about a mechanical rotation centre, the centre being in a horizontal x-y plane perpendicular to an axis of rotation, said first rotating movement having a first offset where the offset is defined with respect to a centreline (51) of the detector, the offset being a shortest distance, of the said centreline between the X-ray source and the X-ray detector, from the said mechanical rotation centre,
change the offset of the imaging between imaging phases,
perform at least one other imaging phase with at least one changed offset (50) to produce second image information of at least part of the object, wherein the mechanical rotation centre in the other imaging phase is moved in the plane relative to the centre of the first rotating movement of the first imaging phase, and
combine projection information, which has been produced in the first phase and the other phase, by image processing carried out by means of an image processing unit for producing three-dimensional image information of the object.

8. A device as claimed in claim 7, wherein the actuator means perform the first scanning imaging cycle of the object by using as offset a predetermined offset (50) and the positioning means change the offset essentially by a width of an imaging area of the X-ray detector for each subsequent imaging phase, a path of projections taken forming a path having a shape of zigzag arcs or arcs within one another on a imaging plane.

9. A device as claimed in claim 7 or 8, wherein the actuator means perform the first scanning imaging cycle of the object by using as offset a predetermined offset (50) and the positioning means change the offset essentially by a width of an imaging area of the X-ray detector for each subsequent imaging phase, a path of projections taken forming a spiral path on a imaging plane.

10. A device as claimed in any of the claims 7-9, wherein the positioning means change positioning of the X-ray source, the collimator and/or the X-ray detector with respect to one another between X-ray imaging phases by displacing the X-ray detector to a new position to provide the offset of at least one subsequent X-ray imaging phase and by directing the radiation at the displaced X-ray detector by changing the positioning of the collimator and/or the X-ray source.

11. A device as claimed in claim 7, wherein the actuator means perform the X-ray imaging of the object by first scanning an image angle area of approximately 360 degrees with an essentially maximal X-ray imaging offset, the positioning means displace the X-ray detector to other side of a line passing through the rotation centre to provide an essentially maximal offset (50) for the at least one subsequent X-ray imaging by scanning the said approximately 360 degree imaging angle area again.

12. A device as claimed in any of the claims 7-11, wherein the positioning means (54) set the offset adjustably for choosing an irradiation area of the scanning X-ray imaging.

13. A device as claimed in any of the claims 7-12, which comprises means for performing, during a single scanning, two or more imaging phases with different offsets (50) of the same object during the same tomographic imaging.

14. A device as claimed in any of the claims 7-13, which comprises means for setting the offset to zero for a symmetrical imaging phase.

15. A software product comprising instructions which, when the product is executed by a computer, cause the X-ray device as claimed in any of the claims 7-14 to perform the method as claimed in claim 1.

16. A software product as claimed in claim 15, wherein the offset is guided to be changed either between separate imaging phases or during an imaging phase so that imaging areas of successive imaging phases are produced into one or more files.

## Patentansprüche

1. Verfahren zum Ausführen von Computertomografie, wobei beim Verfahren
Röntgenstrahlung mit einer Röntgenquelle (41) erzeugt wird, die zu einem Kegelstrahl mittels eines Kollimierungssystems, das mindestens einen Kollimator umfasst, auf ein Objekt (43) kollimiert wird, das abgebildet wird, und die Röntgenstrahlung, die durch das Objekt übertragen worden ist, wird von einem Röntgendetektor (44) aufgenommen, um Bildinformationen des abgebildeten Objektes zu erzeugen, wobei der Detektor einen Breiten- und Bildgebungsbereich hat, und
Röntgenbildgebung wird aus unterschiedlichen Bildgebungswinkeln ausgeführt, indem die Röntgenquelle und der Röntgendetektor relativ zum abgebildeten Objekt bewegt werden, indem sie einer abtastenden Bildgebungsbewegung folgen, wobei die Röntgenquelle und der Röntgendetektor synchron zueinander bewegt werden, ein Kontrollsystem bewegt den Kollimator für das Kollimierungssystem synchron mit der Bewegung des Röntgendetektors relativ zur Röntgenquelle, und Projektionen des Objektes werden in gewünschten Bildgebungswinkelintervallen aufgenommen,
wobei bei diesem Verfahren:
eine erste Bildgebungsphase durchgeführt wird durch Scannen von mindestens einem Teil des Objektes, das abgebildet werden soll, durch Folgen einem ersten Bogen einer ersten Drehbewegung, um erste Bildinformationen zu erzeugen, wobei die erste Drehbewegung eine Rotation um ein mechanisches Rotationszentrum ist, wobei die Mitte in einer horizontalen XY-Ebene senkrecht zu einer Drehachse ist, wobei die erste Drehbewegung eine erste Versetzung hat, wobei die Versetzung (50) mit Bezug auf eine Mittellinie (51) des Detektors definiert wird, wobei die Versetzung eine kürzeste Distanz der Mittellinie zwischen der Röntgenquelle und dem Röntgendetektor von der mechanischen Rotationsmitte ist, die Versetzung der Bildgebung wird zwischen den Bildgebungsphasen geändert,
mindestens eine weitere Bildgebungsphase wird ausgeführt mit mindestens einer geänderten Versetzung (50), um zweite Bildinformationen von mindestens einem Teil des Objektes zu erzeugen, wobei das mechanischer Rotationszentrum in der anderen Bildgebungsphase in der Ebene relativ zur Mitte der ersten Rotationsbewegung der ersten Bildgebungsphase bewegt wird, und
Projektionsinformationen, die in der ersten Phase und in der anderen Phase erzeugt wurden, werden durch Bildverarbeitung kombiniert, die mittels einer Bildverarbeitungseinheit zum Erzeugen der dreidimensionalen Bildinformationen des Objektes ausgeführt wird.

2. Verfahren nach Anspruch 1, wobei eine erste oder letzte Scanning-Bildgebungsphase ausgeführt wird durch Verwendung einer vorgegebenen Versetzung, zum Beispiel eine volle oder keine Versetzung, d. h. symmetrische Versetzung, nachdem oder bevor die Versetzung wesentlich geändert wird durch eine Breite eines Bildgebungsbereichs des Röntgendetektors für jede nachfolgende Bildgebungsphase, wenn ein Pfad von Projektionen, die aufgenommen wurden, sich erhöht oder sich verringert in einer Bildgebungsebene in einer Form, die zum Beispiel spiralförmig, mäanderförmig oder ein separater Bogen relativ zur Mitte ist.

3. Verfahren nach Anspruch 1 oder 2, wobei eine Positionierung der Röntgenquelle, des Kollimators und/oder des Röntgendetektors relativ zueinander geändert wird zwischen den Röntgen-Bildgebungsphasen durch Verschieben des Röntgendetektors auf eine neue Position, um für eine nächste Röntgen-BildgebungsVersetzung zu sorgen, und durch Lenken der Strahlung auf den verschobenen Röntgendetektor durch Ändern einer Positionierung des Kollimators und/oder der Röntgenquelle.

4. Verfahren nach einem der Ansprüche 1-3, wobei zum Wählen eines Bestrahlungsbereichs der Scanning-Röntgenbildgebung eine einstellbare Versetzungseinstellung verwendet wird.

5. Verfahren nach einem der Ansprüche 1-4, wobei Daten, die während der Scanningzyklen gesammelt wurden, vor der 3D-Rekonstruktion zusammengestellt werden.

6. Verfahren nach einem der Ansprüche 1-5, wobei Teilrekonstruktionen, die in einer endgültigen Rekonstruktion verwendet werden, aus den Daten erzeugt werden, die während der Scanningzyklen vor der endgültigen 3D-Rekonstruktion gesammelt wurden.

7. Vorrichtung zum Ausführen der Computertomografie, umfassend
eine Röntgenquelle (41) zum Erzeugen von Röntgenstrahlung,
ein Kollimierungssystem, das mindestens einen Kollimator zum Kollimieren von Röntgenstrahlung auf ein Objekt (43) umfasst, das abgebildet werden soll,
einen Röntgendetektor (44) zum Aufnehmen der Röntgenstrahlung, die durch das Objekt gesendet wurde, um Bildinformationen vom Objekt zu erzeugen, das abgebildet wird, wobei der Detektor eine Breiten- und Bildgebungsfläche hat,
Stützmittel (54) zum Abstützen der Röntgenquelle und des Röntgendetektors während der Bilderzeugung in Positionen auf entgegengesetzten Seiten des Objektes,
Betätigungsmittel zum Drehen der Stützmittel, und Positionierungsmittel (54) zum Ändern einer Röntgen-Bildgebungs-Versetzung (50),
wobei die Vorrichtung dafür ausgelegt ist,
die Röntgenquelle und den Röntgen-Detektor relativ zum Objekt zu bewegen, das abgebildet wird, durch Folgen einer Scanning-Bildgebungsbewegung, wobei die Röntgenquelle und der Röntgendetektor synchron zueinander bewegt werden, ein Kontrollsystem bewegt den Kollimator für das Kollimierungssystem synchron mit der Bewegung des Röntgendetektors relativ zur Röntgenquelle, und Projektionen des Objektes werden unter gewünschten Bildgebungs-Winkelintervallen aufgenommen,
eine erste Bildgebungsphase auszuführen durch Scannen von mindestens einem Teil des Objektes, das abgebildet werden soll, durch Folgen eines ersten Bogens einer ersten Rotationsbewegung, um erste Bildinformationen zu erzeugen, wobei die erste Rotationsbewegung eine Rotation um ein mechanisches Rotationszentrum ist, wobei das Zentrum in einer horizontalen x-y-Ebene senkrecht zu einer Rotationsachse ist, wobei die erste Rotationsbewegung eine erste Versetzung hat, wobei die Versetzung mit Bezug auf eine Mittellinie (51) des Detektors definiert ist, wobei die Versetzung eine kürzeste Distanz der Mittellinie zwischen der Röntgenquelle und dem Röntgendetektor vom mechanischen Rotationszentrum ist,
die Versetzung der Bildgebung zwischen Bildgebungsphasen zu ändern,
mindestens eine andere Bildgebungsphase mit mindestens einer geänderten Versetzung (50) auszuführen, um zweite Bildinformationen von mindestens einem Teil des Objektes zu erzeugen, wobei das mechanische Rotationszentrum in der anderen Bildgebungsphase in der Ebene relativ zum Zentrum der ersten Rotationsbewegung der ersten Bildgebungsphase bewegt wird, und
Projektionsinformationen zu kombinieren, die in der ersten Phase und in der anderen Phase erzeugt worden sind, durch Bildverarbeitung, die mittels einer Bildverarbeitungseinheit zum Erzeugen von dreidimensionalen Bildinformationen des Objektes ausgeführt wird.

8. Vorrichtung nach Anspruch 7, wobei die Betätigungsmittel den ersten Scanning-Bildgebungszyklus des Objektes ausführen, indem als Versetzung eine vorgegebene Versetzung (50) verwendet wird und die Positionierungsmittel die Versetzung im Wesentlichen um eine Breite eines Bildgebungsbereichs des Röntgendetektors für jede nachfolgende Bildgebungsphase ändern, wobei ein Pfad von Projektionen, die aufgenommen wurden, einen Pfad bilden, der die Form eines Zickzackbogens oder von ineinander angeordneten Bögen auf einer Bildgebungsebene hat.

9. Vorrichtung nach Anspruch 7 oder 8, wobei die Betätigungsmittel den ersten Scanning-Bildgebungszyklus des Objektes durch Verwendung einer vorgegebenen Versetzung (50) als Versetzung ausführen und die Positionierungsmittel die Versetzung im Wesentlichen um eine Breite eines Bildgebungsbereichs des Röntgendetektors für jede nachfolgende Bildgebungsphase ändern, wobei ein Pfad von Projektionen, die aufgenommen wurden, einen Spiralpfad auf einer Bildgebungsebene bilden.

10. Vorrichtung nach einem der Ansprüche 7-9, wobei die Positionierungsmittel die Positionierung der Röntgenquelle, des Kollimators und/oder des Röntgendetektors relativ zueinander zwischen Röntgen-Bildgebungsphasen ändern durch Verschieben des Röntgendetektors auf eine neue Position, um für die Versetzung von mindestens einer nachfolgenden Röntgen-Bildgebungsphase zu sorgen und durch Lenken der Strahlung auf den verschobenen Röntgendetektor durch Ändern der Positionierung des Kollimators und/oder der Röntgenquelle.

11. Vorrichtung nach Anspruch 7, wobei die Betätigungsmittel die Röntgen-Bildgebung des Objektes dadurch ausführen, dass zuerst ein Bildwinkelbereich von ca. 360 Grad mit einer im Wesentlichen maximalen Röntgen-Bildgebungsversetzung gescannt wird, die Positionierungsmittel verschieben den Röntgendetektor auf eine andere Seite einer Linie, die durch das Rotationszentrum verläuft, um für eine im Wesentlichen maximale Versetzung (50) für die mindestens eine nachfolgende Röntgen-Bildgebung zu sorgen durch erneutes Scannen des annähernd 360 Grad-Bildgebungswinkelbereichs.

12. Vorrichtung nach einem der Ansprüche 7-11, wobei die Positionierungsmittel (54) die Versetzung einstellbar einrichten zum Wählen eines Bestrahlungsbereichs der Scanning-Röntgen-Bildgebung.

13. Vorrichtung nach einem der Ansprüche 7-12, die Mittel zum Ausführen, während eines einzelnen Scanningvorgangs, von zwei oder mehr Bildgebungsphasen mit unterschiedlichen Versetzungen (50) desselben Objektes während derselben tomografischen Bildgebung umfassen.

14. Vorrichtung nach einem der Ansprüche 7-13, die Mittel zum Einstellen der Versetzung auf null für eine symmetrische Bildgebungsphase umfassen.

15. Softwareprodukt, das Anweisungen umfasst, die bei Ausführung des Produktes durch einen Computer die Röntgenvorrichtung nach einem der Ansprüche 7-14 veranlassen, das Verfahren auszuführen, wie in Anspruch 1 beansprucht.

16. Softwareprodukt nach Anspruch 15, wobei die Versetzung so geführt wird, dass sie entweder zwischen getrennten Bildgebungsphasen oder während einer Bildgebungsphase geändert wird, sodass die Bildgebungsbereiche von aufeinanderfolgenden Bildgebungsphasen in einer oder mehrerer Dateien erzeugt werden.

## Revendications

1. Procédé pour effectuer une tomographie informatisée, dans lequel procédé
un rayonnement X est produit avec une source de rayons X (41), qui est collimatée en un faisceau conique au moyen d'un système de collimation comprenant au moins un collimateur vers un objet (43) étant imagé, et le rayonnement X qui a été transmis à travers l'objet est reçu avec un détecteur de rayons X (44) pour produire une information d'image de l'objet étant imagé, ledit détecteur ayant une largeur et une zone d'imagerie et
une imagerie à rayons X est effectuée à partir d'angles d'imagerie différents en déplaçant la source de rayons X et le détecteur de rayons X par rapport à l'objet étant imagé en suivant un mouvement d'imagerie par balayage, où la source de rayons X et le détecteur de rayons X sont déplacés de manière synchrone l'un par rapport à l'autre, un système de commande déplace le collimateur pour le système de collimation de manière synchrone avec le mouvement du détecteur de rayons X par rapport à la source de rayons X et les projections de l'objet sont prises à des intervalles d'angles d'imagerie désirés,
dans lequel procédé :
une première phase d'imagerie est effectuée en balayant au moins une partie de l'objet à imager en suivant un premier arc d'un premier mouvement de rotation afin de produire une première information d'image, ledit premier mouvement de rotation étant une rotation autour d'un centre de rotation mécanique, le centre étant dans un plan X-Y horizontal perpendiculaire à un axe de rotation, ledit premier mouvement de rotation ayant un premier décalage où le décalage (50) est défini par rapport à une ligne centrale (51) du détecteur, le décalage étant une distance la plus courte de ladite ligne centrale entre la source de rayons X et le détecteur de rayons X, par rapport audit centre de rotation mécanique, le décalage de l'imagerie est changé entre des phases d'imagerie,
au moins une autre phase d'imagerie est effectuée avec au moins un décalage changé (50) pour produire une seconde information d'image et au moins une partie de l'objet, dans lequel le centre de rotation mécanique dans l'autre phase d'imagerie est déplacé dans le plan par rapport au centre du premier mouvement de rotation de la première phase d'imagerie, et
une information de projection, qui a été produite dans la première phase et l'autre phase, est combinée par un traitement d'image exécuté au moyen d'une unité de traitement d'image pour produire une information d'image tridimensionnelle de l'objet.

2. Procédé selon la revendication 1, dans lequel une première ou dernière phase de l'imagerie par balayage est exécutée en utilisant comme décalage un décalage prédéterminé, par exemple un décalage total ou aucun décalage, c'est-à-dire un décalage symétrique, avant ou après lequel le décalage est changé essentiellement par une largeur d'une zone d'imagerie du détecteur de rayons X, pour chaque phase d'imagerie subséquente, lorsque les trajectoires des projections prises augmentent ou diminuent sur un plan d'imagerie dans une forme qui est par exemple en spirale, en méandres ou un axe séparé par rapport au centre.

3. Procédé selon la revendication 1 ou 2, dans lequel un positionnement de la source de rayons X, du collimateur et/ou du détecteur de rayons X est changé les uns par rapport aux autres entre les phases d'imagerie par rayons X en déplaçant le détecteur de rayons X vers une nouvelle position afin de fournir un décalage d'imagerie par rayons X suivant et en dirigeant le rayonnement au niveau du détecteur de rayons X déplacé en changeant un positionnement du collimateur et/ou de la source de rayons X.

4. Procédé selon une quelconque des revendications 1-3, dans lequel est utilisé un réglage de décalage ajustable pour choisir une zone d'irradiation de l'imagerie par un rayons X par balayage.

5. Procédé selon une quelconque des revendications 1-4, dans lequel des données collectées pendant les cycles de balayage sont collectées conjointement avant une reconstruction 3D.

6. Procédé selon une quelconque des revendications 1-5, dans lequel des reconstructions partielles, qui sont utilisés dans une reconstruction finale, sont produites des données collectées pendant les de balayage avant la reconstruction en 3D finale.

7. Dispositif pour mettre en œuvre une tomographie informatisée, comprenant :
une source de rayons X (41) pour produire un rayonnement X ;
un système de collimation comprenant au moins un collimateur pour collimater un rayonnement X vers un objet (43) étant imagé ;
un détecteur de rayons X (44) pour recevoir le rayonnement X qui est transmis à travers l'objet afin de produire une information d'image de l'objet étant imagé, ledit détecteur ayant une largeur et une zone d'imagerie ;
un moyen de soutien (54) pour soutenir la source de rayons X et le détecteur de rayons X pendant l'imagerie dans des positions sur le côté opposé de l'objet ;
un moyen d'actionneur pour tourner rotativement le moyen de soutien, et
un moyen de positionnement (54) pour changer un décalage d'imagerie par rayons X (50),
lequel dispositif est adapté pour
déplacer la source de rayons X et le détecteur de rayons X par rapport à l'objet étant imagé en suivant un mouvement d'imagerie par balayage ou la source de rayons X et le détecteur de rayons X sont déplacés de manière synchrone l'un par rapport à l'autre, un système de commande déplace le collimateur pour le système de collimation de manière synchrone avec le mouvement du détecteur de rayons X par rapport à la source de rayons X et des projections de l'objet sont prises à des intervalles d'angle d'imagerie désirés,
exécuter une première phase d'imagerie en balayant au moins une partie de l'objet à imager en suivant un premier arc d'un premier mouvement de rotation pour produire une première information d'image, ledit premier mouvement de rotation étant une rotation autour d'un centre de rotation mécanique, le centre étant dans un plan X-Y horizontal perpendiculaire à un axe de rotation, ledit premier mouvement de rotation ayant un premier décalage où le décalage est défini par rapport à ligne centrale (51) du détecteur, le décalage étant une distance la plus courte de ladite ligne centrale entre la source de rayons X et le détecteur de rayons X, par rapport audit centre de rotation mécanique,
changer le décalage de l'imagerie entre des phases d'imagerie,
effectuer au moins une autre phase d'imagerie avec au moins un décalage changé (50) pour produire une seconde information d'image au moins une partie de l'objet, dans lequel le centre de rotation mécanique dans l'autre phase d'imagerie est déplacé dans le plan par rapport au centre du premier mouvement de rotation de la première phase d'imagerie, et
combiner l'information de projection, qui a été produite dans la première phase et l'autre phase, par un traitement d'image exécuté au moyen d'unité de traitement d'images pour produire une information d'image tridimensionnelle de l'objet.

8. Dispositif selon la revendication 7, dans lequel le moyen d'actionneur exécute le premier cycle d'imagerie par balayage de l'objet en utilisant comme décalage un décalage déterminé (50) et le moyen de positionnement change le décalage essentiellement par une largeur d'une zone d'imagerie du détecteur de rayons X pour chaque phase d'imagerie subséquente, une trajectoire des projections prises formant une trajectoire ayant une forme d'arcs en zigzag ou d'arcs à l'intérieur l'un de l'autre sur un plan d'imagerie.

9. Dispositif selon la revendication 7 ou 8, dans lequel le moyen d'actionneur exécute le premier cycle d'imagerie par balayage de l'objet en utilisant comme décalage un décalage prédéterminé (51) et le moyen de positionnement change le décalage essentiellement par une largeur d'une zone d'imagerie du détecteur de rayons X pour chaque phase d'imagerie subséquente, une trajectoire des projections prises formant une trajectoire en spirale sur un plan d'imagerie.

10. Dispositif selon une quelconque des revendications 7-9, dans lequel le moyen de positionnement change le positionnement de la source de rayons X, du collimateur et/ou du détecteur de rayons X les uns par rapport aux autres entre des phases d'imagerie par rayons X en déplaçant le détecteur de rayons X vers une nouvelle position afin de fournir le décalage d'au moins une phase d'imagerie par rayons X subséquente et en dirigeant le rayonnement au niveau du détecteur de rayons X déplacé en changeant le positionnement du collimateur et/ou de la source de rayons X.

11. Dispositif selon la revendication 7, dans lequel le moyen d'actionneur met en œuvre l'imagerie par rayons X de l'objet en balayant d'abord un périmètre d'angle d'image d'approximativement 360 degrés avec un décalage d'imagerie par rayons X essentiellement maximal, le moyen de positionnement déplace le détecteur de rayons X vers l'autre côté d'une ligne passant à travers le centre de rotation pour fournir un décalage essentiellement maximal (50) pour au moins une imagerie par rayons X subséquente en balayant de nouveau ledit périmètre d'angle d'imagerie d'approximativement 360 degrés.

12. Dispositif selon une quelconque des revendications 7-11, dans lequel le moyen de positionnement (54) règle le décalage de manière ajustable en choisissant une zone d'irradiation de l'imagerie par rayons X par balayage.

13. Dispositif selon une quelconque des revendications 7-12, qui comprend un moyen pour effectuer, pendant un balayage unique, deux ou plus phases d'imagerie avec des décalages différents (50) du même objet pendant la même imagerie tomographique.

14. Dispositif selon une quelconque des revendications 7-13, qui comprend un moyen pour régler le décalage à zéro pour une phase d'imagerie symétrique.

15. Produit logiciel comprenant des instructions qui, lorsque le produit est exécuté par un ordinateur, amène le dispositif à rayons X selon une quelconque des revendications 7-14 à mettre en œuvre le procédé selon la revendication 1.

16. Produit logiciel selon la revendication 15, dans lequel le décalage est guidé afin d'être changé soit entre des phases imagerie séparées soit pendant une phase d'imagerie de sorte que des zones d'imagerie des phases d'imagerie successives soient produites en un ou plusieurs fichiers.
